# EUROPÄISCHE PATENTSCHRIFT

(11) **EP 1 090 611 B1**
(45) Veröffentlichungstag und Bekanntmachung des Hinweises auf die Patenterteilung: **20.07.2005**
(21) Anmeldenummer: 00118488.6
(22) Anmeldetag: 25.08.2000
(51) Int. Cl.: A61F 5/00, A61F 5/34

(54) **Polster mit weichelastischem Bereich**
Cushion having a flexible region
Coussin comprenant une partie souple

(30) Priorität: 05.10.1999 DE 29917514 U
(43) Veröffentlichungstag der Anmeldung: 11.04.2001
(73) Patentinhaber: Thämert Orthopädische Hilfsmittel GmbH & Co. KG, 30938 Burgwedel/Grossburgwedel (DE)
(72) Erfinder: Littmann, Alexander, 30916 Isernhagen (DE); Schneider-Nieskens, Reinhold, 21365 Adendorf (DE); Blau, Hans Georg, 30938 Burgwedel (DE)
(74) Vertreter: Siekmann, Gunnar, Dipl.-Phys.

(56) Entgegenhaltungen:
- EP-A- 0 717 941
- EP-A- 0 934 732
- DE-U- 8 700 681
- FR-A- 2 712 487
- US-A- 4 287 885
- US-A- 5 027 801

## Beschreibung

Die Erfindung betrifft ein Polster mit wenigstens einem weichelastischen Bereich, insbesondere ein Filzpolster mit einer Silikonpelotte, zur Verwendung mit orthopädischen Hilfsmitteln, bei dem der weichelastische Bereich lösbar an dem Polster angeordnet ist.

Es ist bekannt, derartige Polster in orthopädischen Hilfsmitteln, beispielsweise in Epicondylitisspangen einzusetzen. Dazu wird ein an die Form der Epicondylitisspange angepaßtes Filzpolster verwendet, das in seinen Endbereichen Silikonpunkte aufweist, die sich in besonderer Weise an die Haut des Benutzers des orthopädischen Hilfsmittels anlegen und ein Verrutschen des Polsters verhindern. Dabei werden üblicherweise in die Filzpolster Ausnehmungen geschnitten und weichelastische Bereiche bzw. Silikongießlinge entsprechender Größe in den Ausnehmungen verklebt.

Ein gattungsgemäßes Polster ist aus der US 5,027,801 bekannt. Hier ist eine Gelpolsteranordnung lösbar an einer Nackenstütze befestigt. Die Gelpolsteranordnung ist dabei eine Gesamtanordnung aus einem Silikongießling mit daran befestigten Deckschichten. Diese Gesamtanordnung wird lösbar an bzw. auf der Nackenstütze befestigt.

Aus der DE 87 00 681 U ist es bekannt, in orthopädische Einlagen Polster aus Silikonkautschuk in Vertiefungen einzukleben oder in situ einzugießen.

Der Erfindung liegt die Aufgabe zugrunde, ein Polster der eingangs genannten Art zu schaffen, das besonders lange verwendbar ist.

Diese Aufgabe wird mit einem Polster mit den Merkmalen des Patentanspruchs 1 gelöst. Vorteilhafte Weiterbildungen der Erfindung sind in den Unteransprüchen angegeben.

Nach dem Grundgedanken der Erfindung ist der weichelastische Bereich als Silikongießling ausgebildet, weist das Polster eine zu dem Silikongießling korrespondierende Ausnehmung auf und weist der Silikongießling mindestens ein Halteelement auf. Auf diese Weise kann Material eingespart werden, da der Bestandteil des Polsters, nämlich entweder das Polster selbst oder der weichelastische Bereich, der einem geringeren Verschleiß unterliegt, weiterverwendet werden kann. Wenn also der weichelastische Bereich, insbesondere die Silikonpelotte, durch häufige Benutzung unansehnlich geworden ist und nicht mehr verwendet werden soll, so kann der weichelastische Bereich aus dem Polster entnommen werden und durch einen neuen ersetzt werden. Umgekehrt kann ebenso der Polsterbereich ausgewechselt werden, falls dieser einem größeren Verschleiß unterliegt. Grundsätzlich sind sehr viele verschiedene Befestigungsmöglichkeiten denkbar, mit denen das Polster an dem weichelastischen Bereich angeordnet werden kann. Erfindungsgemäß weist der weichelastische Bereich ein Halteelement auf, mit dem der weichelastische Bereich das Polster in Eingriff nimmt. Grundsätzlich ist es auch denkbar, den weichelastischen Bereich größenmäßig so auszubilden, daß er in eine Ausnehmung des Polsters eingeschoben wird und dort allein durch eine Klemmwirkung verbleibt. Dazu müßte der weichelastische Bereich genau so groß wie die entsprechende Ausnehmung oder geringfügig größer als die entsprechende Ausnehmung ausgebildet werden.

Bevorzugt ist es jedoch, den weichelastischen Bereich, der günstigerweise als Silikongießling ausgebildet ist, so auszubilden, daß das Halteelement über die Abmessungen des übrigen weichelastischen Bereichs hinaussteht. Das Halteelement ist dabei günstigerweise als Halteflansch ausgebildet, der an mindestens zwei Stellen des weichelastischen Bereichs vorgesehen ist und günstigerweise am oberen Rand des weichelastischen Bereichs angeordnet ist, so daß der weichelastische Bereich in eine korrespondierende Ausnehmung des Polsters eingelegt werden kann und die am oberen Rand vorgesehenen Halteflansche auf dem Polster aufliegen. Dazu ist der weichelastische Bereich um die Dicke des Halteflansches dicker als das Polster ausgebildet. Der Halteflansch ist in einer bevorzugten Ausführungsform umlaufend und durchgängig ausgebildet, so daß die Haltewirkung im gesamten weichelastischen Bereich gegeben ist. Der Halteflansch ist günstigerweise auf der körperabgewandten Seite angeordnet, so daß auf der dem Körper zugewandten Seite ein bündiger oder ein anderer Abschluß des weichelastischen Bereichs in dem Polster gegeben ist. Günstig ist es auch, den weichelastischen Bereich und den Halteflansch einstückig auszubilden, da auf diese Weise die Herstellung vereinfacht und dadurch besonders kostengünstig möglich ist.

In einer anderen besonders bevorzugten Ausführungsform der Erfindung ist als Halteelement ein Klettelement vorgesehen. Dies kann entweder ein Klettband oder Hakenband oder ein korrespondierender Flausch sein. Wenn an dem weichelastischen Bereich ein Klettband vorgesehen ist, so ist dann an dem Polster bevorzugt ein Flausch angeordnet. Auch die umgekehrte Ausführungsform, nämlich das an dem weichelastischen Bereich ein Flausch angeordnet ist, ist möglich. Bevorzugt ist das Klettelement über den weichelastischen Bereich vorstehend ausgebildet, so daß das Klettelement mit einem korrespondierenden Belag auf der Rückseite des Polsters in Eingriff gebracht werden kann. Der weichelastische Bereich kann auf diese Weise in eine Ausnehmung des Polsters eingelegt und mit dem Klettelement an dem Polster lösbar befestigt werden. Bei Bedarf kann der weichelastische Bereich dann losgeklettet und durch einen neuen weichelastischen Bereich ausgetauscht werden. Alternativ ist es auch möglich, das Klettelement rückseitig an dem weichelastischen Bereich anzuordnen. Die Klettverbindung wird dann zu dem orthopädischen Hilfsmittel hergestellt, das mit dem Polster verwendet wird.

Das Klettelement kann auf verschiedene Weisen mit dem weichelastischen Bereich verbunden sein. Bevorzugt ist das Klettelement mit dem weichelastischen Bereich verwachsen. Das bedeutet, daß das Klettelement mit dem weichelastischen Bereich, insbesondere mit dem Silikon eine etwa 1 mm starke Grenzschicht bildet, in der der weichelastische Bereich in das Klettelement eingezogen ist. Alternativ ist es auch möglich, daß das Klettelement auf dem weichelastischen Bereich aufgeklebt ist.

Zur Herstellung eines solchen Polsters ist es erfindungsgemäß vorgesehen, daß zur Bildung eines Gießlings in ein Formwerkzeug ein fließfähiges Material eingefüllt wird, daß das Klettelement mit dem noch fließfähigen Material in Kontakt gebracht wird und daß das fließfähige Material dann ausvulkanisiert wird und dabei mit dem Klettelement verwächst. Es ist also entscheidend, daß das Klettelement mit dem noch flüssigen Werkstoff, insbesondere dem noch flüssigen Silikon, in Kontakt gebracht wird, bevor dieses ausvulkanisiert ist. Dabei muß die Viskosität des Materials und die Vulkanisationszeit so gewählt werden, daß das fließfähige Material mit dem aufgebrachten Teil, insbesondere dem Klettelement verwächst, dieses aber nicht vollständig durchdringt. Alternativ kann das Klettelement auf dem weichelastischen Bereich aufgeklebt sein. Als Kleber können dabei Reaktionsklebstoffe, Lösemittelklebstoffe oder Schmelzklebstoffe, zum Beispiel Schmelzkleberfolien, dienen.

Nachfolgend wird die Erfindung anhand eines in der Zeichnung dargestellten bevorzugten Ausführungsbeispiels weiter erläutert. Im einzelnen zeigen die schematischen Darstellungen in:
Fig. 1: eine Draufsicht auf ein Polster mit weichelastischem Bereich;
Fig. 2: eine Seitenansicht eines Polster mit einem weichelastischen Bereich gemäß Fig. 1;
Fig. 3: eine Querschnittsansicht eines weichelastischen Bereichs während des Herstellungsverfahrens;
Fig. 4: einen Querschnitt durch ein Polster mit einem gemäß Fig. 3 hergestellten weichelastischem Bereich;
Fig. 5: einen Querschnitt durch eine alternative Ausführungsform eines erfindungsgemäßen weichelastischen Bereichs während der Herstellung;
Fig. 6: eine Darstellung gemäß Fig. 5 in einem fortgeschrittenen Verfahrensstadium und
Fig. 7: einen Querschnitt durch ein Polster mit einer weiteren Ausführungsform des weichelastischen Bereichs.

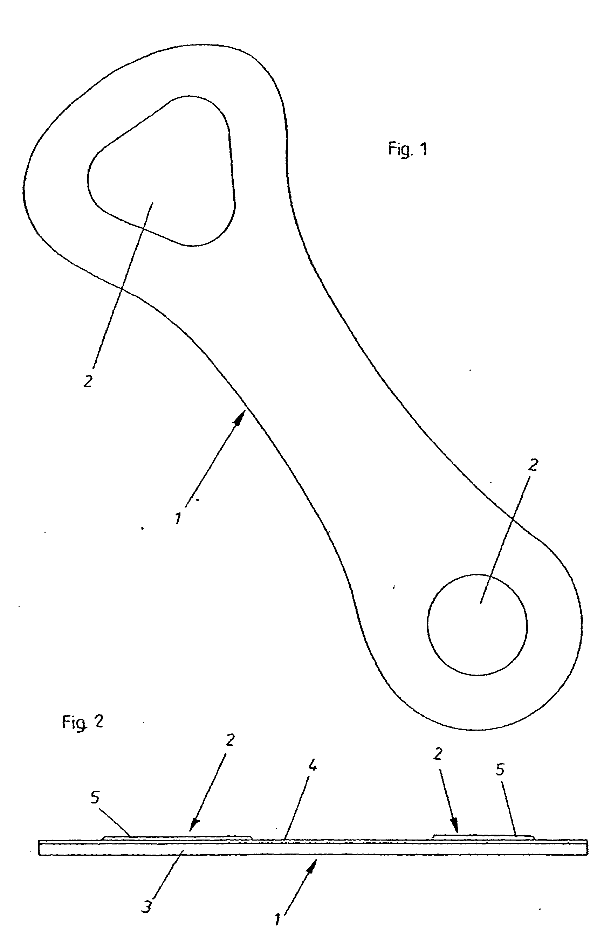

In Fig. 1 ist ein Polster 1 dargestellt, das zur Verwendung in einer Epicondylitisspange bestimmt ist. Das Polster ist in seiner Form an die Form der Epicondylitisspange angepaßt, und in den erweiterten Endbereichen sind weichelastische Einlagen 2 angeordnet, die auf der Haut liegen und ein Verrutschen des Polsters 1 verhindern.

In Fig. 2 ist das erfindungsgemäß hergestellte Polster 1 in einer Seitenansicht dargestellt. Das Polster 1 besteht in seinem unteren Bereich aus einer Filzschicht 3, auf der ein Flausch 4 angeordnet ist, der so ausgebildet ist, daß er mit einem Hakenband verklettbar ist. Die weichelastischen Bereiche 2 können so ausgebildet sein, daß sie ein Halteelement 5 aufweisen, das das Polster 1 überragt. Das Halteelement 5 ist auf der dem Körper abgewandten Seite des Polsters 1 angeordnet.

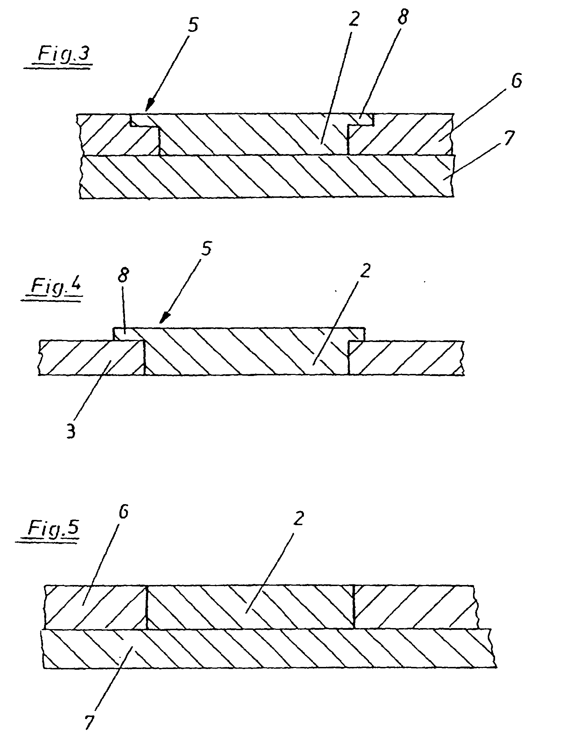

In Fig. 3 ist ein Querschnitt durch einen weichelastischen Bereich 2 während eines Herstellungsverfahrens dargestellt. Dabei wird ein Formwerkzeug 6 verwendet, das auf einer Heizplatte 7 aufliegt. In dieses Formwerkzeug 6 wird ein flüssiges Material, insbesondere flüssiges Silikon, eingegeben, das in dem Formwerkzeug vulkanisiert und die gezeigte Gestalt annimmt. Die Vulkanisationsdauer oder Vulkanisationszeit hängt von der Temperatur, die durch die Heizplatte 7 gesteuert wird, dem Katalysatorgehalt und der ursprünglichen Konsistenz bzw. der Zähigkeit des eingefüllten Materials ab. Der weichelastische Bereich 2 wird einstückig mit einem umlaufenden Halteflansch 8 hergestellt, der in einer entsprechenden Ausnehmung des Formwerkzeugs 2 gebildet ist. Der Halteflansch 8 verläuft am oberen Randbereich des weichelastischen Bereichs 2.

In Fig. 4 ist ein Querschnitt durch einen Bereich eines erfindungsgemäßen Polsters dargestellt, in dem der weichelastische Bereich 2 lösbar in einem Filzpolster 3 angeordnet ist. Der weichelastische Bereich 2 kann also durch den auf dem Polster 3 aufliegenden Halteflansch 8 eingelegt und wieder entnommen werden. Eine besonders sichere Handhabung ist dann gegeben, wenn die Seite des Polsters 3, auf der der Halteflansch 8 aufliegt, dem Benutzer zugewandt angewendet wird. Ein unbeabsichtigtes Lösen des weichelastischen Bereichs 2 ist dann ausgeschlossen. Falls ein durchgängiger Abschluß von Filz 3 und weichelastischem Bereich 2 gewünscht ist, so wird die in der Zeichnung untere Seite dem Benutzer zugewandt. Ein unbeabsichtigtes Herausdrücken des weichelastischen Bereichs 2 aus dem Filz 3 ist auch dann nahezu ausgeschlossen, da das Polster in aller Regel zusammen mit einem orthopädischen Hilfsmittel, beispielsweise einer Epicondylitisspange verwendet wird, an der die Rückseite des weichelastischen Bereichs 2 anliegt. Die Rückseite des weichelastischen Bereichs 2 kann dabei an der Epicondylitisspange befestigt, beispielsweise auf diese aufgeklettet, sein.

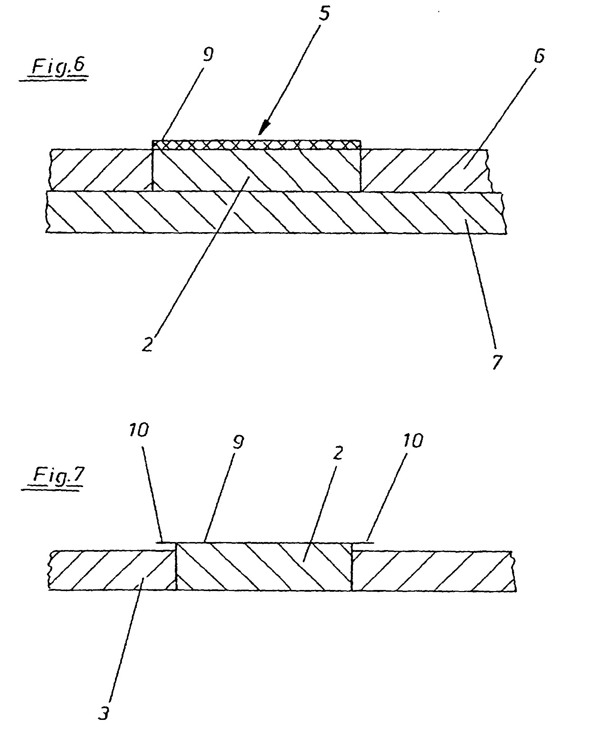

In den Fig. 5 und 6 sind Querschnitte durch ein Polster mit einem weichelastischen Bereich während zweier verschiedener Schritte eines erfindungsgemäßen Verfahrens zu seiner Herstellung dargestellt. In Fig. 5 ist ein Formwerkzeug 6 dargestellt, das auf einer Heizplatte 7 aufliegt und in dem ein weichelastischer Bereich 2 hergestellt wird, indem flüssiges Silikon eingefüllt wird. Bevorzugt sind auf der Heizplatte 7 eine Vielzahl von Formwerkzeugen 6 angeordnet, so daß eine Vielzahl von weichelastischen Bereichen 2 gleichzeitig herstellbar ist. Dies gilt ebenso für die Ausführung des Verfahrens gemäß Fig. 3.

In Fig. 6 ist dargestellt, daß auf den noch flüssigen Gießling gemäß Fig. 5 ein Halteelement 5 aufgelegt wird, das insbesondere von einem Klettelement 9 gebildet ist. Das Klettelement 9 ist entweder ein Flausch oder ein Klettband, so daß es mit einem korrespondierenden Klettband oder Flausch in Klettverbindung gebracht werden kann. Das Klettelement 9 wird dabei so auf den noch flüssigen Gießling aufgebracht, daß das flüssige Material in das Klettelement 9 etwa 1 mm einzieht und verwächst.

Der weichelastische Bereich 2 wird dann mit Hilfe der Heizplatte 7 ausvulkanisiert und der weichelastische Bereich 2 kann dann dem Formwerkzeug 6 entnommen und in einem Polster verwendet werden. Dabei wird das Klettelement 9 gemäß dem gezeigten Ausführungsbeispiel mit dem orthopädischen Hilfsmittel verklettet. Bevorzugt kann der so hergestellte weichelastische Bereich auch mit Polstern verwendet werden, die keine Ausnehmungen, sondern einen zu dem Klettelement 9 korrespondierenden Abschnitt aufweisen. Der weichelastische Bereich wird dann direkt auf das Polster geklettet und steht etwas über dieses hoch. Alternativ besteht auch die Möglichkeit, ein Klettelement 9 zu verwenden, das in seinen Außenabmessungen über die Ränder des weichelastischen Bereichs 2 hinaussteht.

Ein Querschnitt durch einen solch weichelastischen Bereich in einem Filz 3 ist in Fig. 7 dargestellt. Das Klettelement 9 steht umlaufend oder zumindest an zwei bis drei Stellen mit einem Überstand 10 über den weichelastischen Bereich 2 über. Dieser Überstand 10 kann mit seinem nach unten ausgerichteten Klettelement mit dem Filz 3 bzw. einem entsprechenden Flausch oder Klettband, das auf dem Filzelement 3 angeordnet ist, verklettet werden. Dadurch kann der weichelastische Bereich 2 lösbar in dem Polster befestigt werden.

## Patentansprüche

1. Polster mit wenigstens einem weichelastischen Bereich, insbesondere ein Filzpolster mit einer Silikonpelotte, zur Verwendung mit orthopädischen Hilfsmitteln, bei dem der weichelastische Bereich lösbar an dem Polster angeordnet ist,
**dadurch gekennzeichnet,**
**daß** der weichelastische Bereich (2) als Silikongießting ausgebildet ist,
**daß** das Polster eine zu dem Silikongießling korrespondierende Ausnehmung aufweist und
**daß** der Silikongießling mindestens ein Halteelement (5) aufweist.

2. Polster nach Anspruch 1, **dadurch gekennzeichnet, daß** das Halteelement (5) über die Abmessungen des übrigen weichelastischen Bereichs (2) hinaussteht.

3. Polster nach einem der Ansprüche 1 oder 2, **dadurch gekennzeichnet, daß** der weichelastische Bereich (2) einen Halteflansch (8) aufweist.

4. Polster nach Anspruch 3, **dadurch gekennzeichnet, daß** der Halteflansch (8) umlaufend und durchgängig ausgebildet ist.

5. Polster nach einem der vorhergehenden Ansprüche, **dadurch gekennzeichnet, daß** als Halteelement (5) ein Klettelement (9) vorgesehen ist.

6. Polster nach der Anspruch 5, **dadurch gekennzeichnet, daß** das Klettelement (9) rückseitig an dem weichelastischen Bereich (2) angeordnet ist.

7. Polster nach einem der Ansprüche 5 oder 6, **dadurch gekennzeichnet, daß** das Klettelement (9) mit dem weichelastischen Bereich (2) verwachsen ist.

## Claims

1. A cushion having at least one flexible region, especially a felt cushion with a silicone truss pad, for use with orthopaedic aids, wherein the flexible region is arranged detachably on the cushion,
**characterised in**
**that** the flexible region (2) is constructed as a silicone casting,
**that** the cushion has a recess corresponding to the silicone casting and
**that** the silicone casting has at least one retaining element (5).

2. The cushion according to claim 1, **characterised in that** the retaining element (5) projects beyond the dimensions of the remaining flexible region (2).

3. The cushion according to any one of claims 1 or 2, **characterised in that** the flexible region (2) has a retaining flange (8).

4. The cushion according to claim 3, **characterised in that** the retaining flange (8) is constructed such that it is circumferential and universal.

5. The cushion according to any one of the preceding claims, **characterised in that** a Velcro element (9) is provided as the retaining element (5).

6. The cushion according to claim 5, **characterised in that** the Velcro element (9) is arranged on the back on the flexible region (2).

7. The cushion according to any one of claims 5 or 6, **characterised in that** the Velcro element (9) is adherent to the flexible region (2).

## Revendications

1. Rembourrage avec au moins une zone souple, en particulier rembourrage en feutre avec une pelote en silicone, destiné à être utilisé avec des accessoires orthopédiques, ladite zone souple étant disposée de manière amovible sur le rembourrage,
**caractérisé en ce que** la zone souple (2) est conçue comme une pièce moulée en silicone, **en ce que** le rembourrage comporte un creux correspondant à la pièce moulée en silicone et **en ce que** la pièce moulée en silicone comporte au moins un élément de fixation (5).

2. Rembourrage selon la revendication 1, **caractérisé en ce que** l'élément de fixation (5) dépasse des dimensions du reste de la zone souple (2).

3. Rembourrage selon la revendication 1 ou 2, **caractérisé en ce que** la zone souple (2) comporte une bride de fixation (8).

4. Rembourrage selon la revendication 3, **caractérisé en ce que** la bride de fixation (8) est périphérique et continue.

5. Rembourrage selon l'une des revendications précédentes, **caractérisé en ce qu'**il est prévu comme élément de fixation (5) un élément autoagrippant (9).

6. Rembourrage selon la revendication 5, **caractérisé en ce que** l'élément autoagrippant (9) est disposé avec sa face arrière sur la zone souple (2).

7. Rembourrage selon la revendication 5 ou 6, **caractérisé en ce que** l'élément autoagrippant (9) et la zone souple (2) sont réalisés d'une seule pièce.
